# FASCICULE DE BREVET EUROPEEN

(11) **EP 2 691 057 B1**
(45) Date de publication et mention de la délivrance du brevet: **24.05.2017**
(21) Numéro de dépôt: 12717410.0
(22) Date de dépôt: 29.03.2012
(51) Int. Cl.: A61F 13/00

(54) **PANSEMENT ABSORBANT CICATRISANT, SES UTILISATIONS POUR LES PLAIES CHRONIQUES**
ABSORBIERENDER HEILENDER WUNDVERBAND UND VERWENDUNG DAVON FÜR CHRONISCHE WUNDEN
ABSORBENT CICATRIZATION DRESSING AND USES THEREOF FOR CHRONIC WOUNDS

(30) Priorité: 31.03.2011 FR 1152720
(43) Date de publication de la demande: 05.02.2014
(73) Titulaire: URGO RECHERCHE INNOVATION ET DEVELOPPEMENT, 21300 Chenôve (FR)
(72) Inventeur: PERNOT, Jean-Marc, F-21000 Dijon (FR); AUGUSTE, Stéphane, F-21490 Ruffey-lès-Echirey (FR); LAURENSOU, Christelle, F-21000 Dijon (FR)
(74) Mandataire: Chantraine, Sylvie Hélène
(86) Numéro de dépôt international: PCT/FR2012/050665
(87) Numéro de publication internationale: WO 2012/131263

(56) Documents cités:
- WO-A1-02/03898
- WO-A1-2007/025546

## Description

La présente invention se rapporte à un pansement comprenant une compresse non tissée absorbante et une couche de contact élastomère comprenant des hydrocolloïdes. Ce pansement est très efficace dans le soin des plaies dont on veut favoriser la cicatrisation. Il permet de gérer les exsudats générés par la plaie ainsi que la présence de fibrine, sans qu'il soit nécessaire d'avoir recours à des moyens de détersion complémentaires tels que la chirurgie ou les enzymes.

La cicatrisation naturelle d'une plaie se déroule en trois phases successives, chacune de ces phases étant caractérisée par des activités cellulaires spécifiques: la phase de détersion, la phase de bourgeonnement (ou granulation) et la phase d'épithélialisation. Tout au long du processus de cicatrisation, la plaie produit des exsudats fluides ou visqueux qui doivent être absorbés par le pansement.

Les capacités de détersion naturelle de la plaie peuvent être insuffisantes lorsque le traumatisme est important ou lorsque le patient souffre de pathologies parallèles, comme les pathologies veineuses ou le diabète. Dans ces cas, on observe un allongement considérable de la durée de la phase de détersion conduisant à des plaies chroniques difficiles à soigner, comme par exemple l'ulcère de jambe.

Dans le cas de plaies pour lesquelles le processus de détersion naturelle est insuffisant, il est nécessaire d'éliminer le tissu fibrineux sans perturber la phase de bourgeonnement. L'élimination de ce tissu fibrineux est couramment désignée sous le terme de "détersion assistée" par opposition à la détersion naturelle.

Selon la technique utilisée, la détersion assistée peut être qualifiée de détersion mécanique ou chirurgicale, de détersion enzymatique, de détersion autolytique ou de détersion biologique.

La détersion chirurgicale ou mécanique est une technique rapide qui consiste à découper le tissu fibrineux, soit à l'aide d'un bistouri, de pinces, de ciseaux ou d'une curette de Brock, soit à l'aide d'appareils sophistiqués par jets d'eau sous pression ou excision au laser. Cette technique est réalisée au lit du patient ou en milieu chirurgical selon la gravité de la plaie. Cependant, cette technique est souvent douloureuse et peut entraîner des saignements et parfois même une hémorragie. Elle est alors traumatisante pour le patient. Elle nécessite aussi couramment une médication antalgique au préalable qui allonge la durée des soins.

La détersion autolytique consiste à déposer sur la plaie des pansements absorbants à base de fibres gélifiantes particulières.

Les pansements utilisés pour la détersion sont généralement des non tissés aiguilletés de fibres gélifiantes d'alginate ou de carboxyméthylcellulose (un produit de ce genre est commercialisé sous la référence Aquacel^{®} par exemple). Ces pansements présentent l'inconvénient de manquer de cohésion et de se déliter au fur et à mesure de l'absorption des exsudats, si bien qu'ils laissent des débris dans la plaie et que leur retrait en un seul morceau est impossible. Pour résoudre ce problème, il a été proposé de déposer une couche d'adhésif perforée à la surface du non tissé, de manière à retenir les fibres qui se détachent du non tissé tout en garantissant le passage des exsudats (WO 2002/03898). Le diamètre des perforations proposé est de l'ordre de 5 à 8 mm.

Il a également a été proposé dans le document WO 2007/025546 d'utiliser un non tissé de fibres superabsorbantes, dans lequel les fibres sont aiguilletées de manière à les espacer suffisamment pour que, une fois gonflées, elles continuent de laisser passer les exsudats. En espaçant les fibres, la cohésion du non tissé diminue si bien qu'il devient nécessaire d'empêcher les fibres qui se seraient détachées du non tissé de venir polluer la plaie. La demande WO 2007/025544 prévoit ainsi de déposer par fibérisation des fils de matériau thermoplastique sur le non tissé en formant des boucles dont les points d'intersection sont soudés. Le grammage des fils doit être inférieur à 100 g/m² pour assurer l'absorption initiale rapide des exsudats par la couche absorbante. En effet, il est impératif de ne pas diminuer la vitesse et la capacité d'absorption du non tissé en intercalant une couche intermédiaire entre la plaie et le non tissé. De même cette couche intermédiaire ne doit pas altérer la capacité de détersion autolytique de ces pansements.

Ces pansements sont loin de permettre une détersion optimale. Leur utilisation est souvent associée à la détersion mécanique qui nécessite le passage par des actes traumatisants et douloureux pour le patient. Il serait aussi utile de diminuer ou d'éliminer le recours à cette détersion mécanique.

Il serait donc souhaitable de disposer d'un pansement pour le soin des plaies, notamment des plaies chroniques, qui - malgré la présence d'une couche intermédiaire entre la plaie et le non tissé - n'altère pas la quantité et la vitesse d'absorption des exsudats et les capacités de détersion autolytique du pansement et qui permette de préférence un retrait de la fibrine optimisé pour diminuer ou éliminer le recours à des actes chirurgicaux. Ce pansement devrait également permettre de favoriser la cicatrisation des zones de la plaie dépourvues de fibrine et ne pas altérer, lors de son retrait, la reconstruction des tissus. Enfin, ce pansement devrait être cohésif et ne pas provoquer de douleur lors de son retrait.

La Demanderesse a mis au point un pansement répondant à ces souhaits, lequel pansement comprend un non tissé superabsorbant qui est recouvert, sur la partie venant en regard de la plaie, d'une couche contact particulière. Cette couche permet de garantir le retrait indolore du pansement usagé tout en assurant le retrait de la fibrine. Contre toute attente, l'interposition de cette couche particulière entre la plaie et la compresse non tissée n'a pas diminué la capacité du pansement à absorber les exsudats et sa capacité de détersion autolytique. La Demanderesse a en outre constaté que la fibrine peut s'accrocher au pansement et peut se retirer par blocs quand on ôte le pansement.

La présente invention permet ainsi d'optimiser la cicatrisation des plaies, notamment des plaies chroniques, en proposant pour la première fois un pansement qui favorise simultanément la détersion autolytique et la granulation de la plaie et permet de prendre en charge toutes les parties de la plaie, lesquelles ne sont pas nécessairement au même stade de cicatrisation.

Le pansement de l'invention permet par ailleurs d'absorber très rapidement les exsudats liquides tout en maintenant un environnement humide au niveau de la plaie. Les exsudats sont absorbés par la compresse non tissée, tandis que la couche contact gélifie au contact des exsudats. L'environnement humide créé à la surface de la plaie permet de favoriser la cicatrisation.

Enfin le pansement de l'invention est cohésif et ne se déchire pas quand on le retire.

Dans les structures de pansement de l'invention comprenant l'assemblage d'un non tissé de fibres superabsorbantes et d'une couche destinée à entrer en contact avec la plaie, il convient de garantir une absorption rapide et constante au cours du temps des exsudats et d'éviter la macération de la plaie et de la peau périlésionelle.

C'est l'objet de la présente invention que de fournir un pansement cohésif, constitué d'un non tissé particulier et d'une couche de contact spécifique, qui permet le retrait indolore du pansement tout en assurant l'absorption rapide des exsudats sans altérer la capacité de détersion autolytique du pansement, et de préférence en favorisant le retrait de la fibrine par blocs, ainsi que de préserver l'intégrité des tissus de bourgeonnement et favoriser leur développement, et enfin de garantir une absorption des exsudats rapide, et constante tout au long de son utilisation pour éviter les problèmes de macération.

La couche de contact spécifique est constituée d'une composition élastomère pour rester souple et épouser la forme du corps.

Les pansements de l'invention sont indolores au retrait, et leur action sur la cicatrisation des zones sans fibrine est bénéfique car la couche contact, en gélifiant, n'adhère pas à la plaie et crée un environnement humide favorable. L'absorption de la fibrine et des exsudats par le non tissé est conservée en évitant la diminution de la taille des ouvertures de la couche contact au cours de l'absorption des exsudats.

La présente invention a ainsi pour objet un pansement comprenant :
a- une compresse non tissée absorbante formée d'un mélange de fibres superabsorbantes, de préférence bicomposants, et de fibres non absorbantes thermoliantes, l'ensemble des fibres étant thermolié,
   et,
b- une couche contact recouvrant partiellement la face de la compresse destinée à venir en contact avec la plaie, ladite couche comprenant des ouvertures permettant le passage des exsudats de la plaie et présentant un grammage allant de 110 à 500 g/m², et ladite couche étant formée d'une composition comprenant une matrice élastomérique et des hydrocolloïdes, la proportion d'hydrocolloïdes étant comprise entre 2 et 20% en poids du poids de ladite composition.

Selon un mode de mise en oeuvre préféré de l'invention, le pansement comprend :
a- une compresse non tissée absorbante formée d'un mélange de fibres superabsorbantes bicomposants de type coeur/écorce avec un coeur en polyacrylonitrile et une écorce en polyacrylate et de fibres non absorbantes thermoliantes bicomposants du type coeur/écorce, ledit coeur étant en polyester (polyéthylène téréphtalate PET) et l'écorce étant en polyéthylène, l'ensemble des fibres étant thermolié,
   et,
b- une couche contact recouvrant partiellement la face de la compresse destinée à venir en contact avec la plaie, ladite couche comprenant des ouvertures permettant le passage des exsudats et présentant un grammage allant de 150 à 200 g/m², ladite couche étant formée d'une composition comprenant une matrice élastomérique et des hydrocolloïdes, les hydrocolloïdes représentant de 2 à 20% en poids du poids total de ladite composition, et ladite couche recouvrant, avant d'être exposée aux exsudats de la plaie, entre 55 et 65% de la face de la compresse qui est en regard de la plaie.

Le pansement conforme à la présente invention comprend une couche absorbante formée d'un non tissé obtenu à partir d'un mélange de fibres superabsorbantes et de fibres non absorbantes.

Par l'expression « superabsorbantes », on entend désigner ici des fibres qui présentent une très grande capacité d'absorption des liquides, de préférence supérieure ou égale à 10 g d'eau (ou de solution saline telle que du sérum physiologique) par gramme, de préférence encore supérieure à 20 g d'eau par gramme, et de préférence encore supérieure à 30 g d'eau par gramme.

Selon l'invention, les fibres superabsorbantes sont de préférence constituées de deux matériaux différents. Ces matériaux peuvent être répartis selon une configuration côte-à-côte, ou de préférence selon une configuration coeur-écorce.

Le premier matériau destiné à former une partie externe de la fibre, de préférence l'écorce, doit être apte à former un gel avec les exsudats de la plaie et sera avantageusement formé d'un ou plusieurs polymères réticulés et/ou partiellement réticulés, comme en particulier des polymères de l'acide acrylique et/ou des polymères de sels de l'acide acrylique, notamment l'acrylate de sodium ou d'ammonium.

Le second composant qui formera de préférence le coeur des fibres superabsorbantes, sera de préférence non gélifiant et compatible avec le premier matériau pour garantir la stabilité de la fibre après formation d'un gel par le premier matériau. Il peut être formé de tout type de polymère stable en milieu aqueux et compatible avec le matériau de l'écorce pour conduire à une fibre bicomposante stable.

Avantageusement, ce second matériau est formé de polyacrylonitrile.

Les fibres superabsorbantes ont avantageusement une taille comprise entre 2 et 6 dtex.

Des fibres superabsorbantes pouvant être utilisées dans le cadre de l'invention sont par exemple commercialisées par la société TOYOBO CO LTD sous la dénomination LANSEAL^{®} F.

Les fibres non absorbantes sont des fibres thermoliantes aptes à renforcer et stabiliser la structure tridimensionnelle du non tissé en formant une armature résultant de la liaison de ces fibres entre elles et/ou de ces fibres avec les fibres superabsorbantes.

Ces deuxièmes fibres peuvent être constituées d'une matière thermoplastique unique comme par exemple un polyéthylène, un polypropylène ou un polyester de bas point de fusion.

Avantageusement, ces deuxièmes fibres seront également constituées de deux matériaux différents répartis selon une configuration de type côte-à-côte ou de préférence coeur-écorce.

La longueur de ces fibres peut être de l'ordre de 10 à 100 mm, de préférence de 25 à 75 mm.

Dans le cadre de la présente invention, des fibres non absorbantes thermoliantes bicomposants de type coeur-écorce dans lesquelles le coeur est formé d'un polyester comme en particulier le polyéthylène téréphtalate, et l'écorce est formée de polyéthylène, sont particulièrement préférées.

D'une façon générale, le non tissé formant la compresse absorbante des pansements selon l'invention sera obtenu à partir de mélanges incorporant plus de 50 % en poids, de préférence plus de 60% en poids, de fibres superabsorbantes.

Le ratio massique entre les fibres absorbantes et les fibres non absorbantes thermoliantes peut être compris entre 20/80 et 80/20, de préférence entre 60/40 et 80/20. D'excellents résultats ont été obtenus à l'aide d'un mélange comprenant 30 % en poids de fibres non absorbantes et 70 % en poids de fibres superabsorbantes.

Ce non tissé est généralement obtenu par thermoliage, ou par aiguilletage et thermoliage du mélange de fibres.

L'opération d'aiguilletage permet notamment d'orienter les fibres superabsorbantes dans une direction sensiblement verticale par rapport au plan du non tissé. Cette orientation des fibres permet de réduire la propagation transversale des exsudats absorbés par le pansement contenant ce non tissé et permet donc de diminuer les risques de macération et par conséquent d'altération de la peau périlésionnelle.

L'opération de thermoliage permet d'améliorer la résistance au déchirement du non tissé après absorption, en créant des points d'ancrage entre les fibres du non tissé. Il est nécessaire de renforcer la cohésion du non tissé pour permettre le retrait du pansement usagé sans le déchirer.

L'assemblage des fibres sera réalisé dans des conditions permettant d'obtenir un non tissé présentant une épaisseur comprise entre 0,6 et 3 mm, de préférence de 2 mm, et un grammage compris entre 40 et 400 g/m², de préférence de l'ordre de 185 g/m².

La compresse non tissée peut être fabriquée selon le procédé décrit dans le document GB 2 401 879.

Diverses substances actives peuvent être incorporées dans le non tissé, comme par exemple des substances à activité antimicrobienne, notamment des sels d'argent tels que par exemple le sulfate d'argent, le chlorure d'argent, le nitrate d'argent, la sulfadiazine argentique, des ammoniums quaternaires, le polyhexaméthylène biguanide et la Chlorhexidine. On peut incorporer d'autres substances favorisant la cicatrisation comme par exemple des facteurs de croissance ou des oligosaccharides polysulfatés comme le sel de potassium du sucrose octasulfaté.

On peut également incorporer au non tissé des fibres dotées de propriétés antibactériennes. Par exemple, ces fibres pourront incorporer un métal (argent, cuivre, zinc) ou un autre actif antibactérien. Dans le cas d'un métal, ces fibres peuvent être obtenues de différentes façons: par incorporation du métal dans la matrice polymérique lors de l'extrusion (PP, PET, PA) ou par application du métal dans le spin-finish lors du processus de filature (acrylique, viscose). Le métal utilisé pour fabriquer ces fibres peut être sous forme de sels, de zéolithes, de céramiques ou de nanoparticules.

La composition élastomère contenant des hydrocolloïdes susceptible d'être utilisée pour la fabrication des pansements conformes à l'invention comprend une matrice élastomérique dans laquelle des hydrocolloïdes sont de préférence dispersés de façon homogène.

La couche contact du pansement de l'invention permet avantageusement de ne pas adhérer à la plaie et d'éviter toute douleur au retrait du pansement. En maintenant un milieu humide à la surface de la plaie tout en évitant le contact avec la compresse absorbante chargée d'exsudats, elle améliore la cicatrisation. L'incorporation d'hydrocolloïdes confère à la composition élastomère un caractère hydrophile et favorise la vectorisation d'actifs susceptibles de favoriser le traitement de la plaie.

Ladite composition comprend un ou plusieurs élastomères choisis parmi les polymères séquencés poly(styrène-oléfine-styrène). Les copolymères séquencés utilisés dans le cadre de l'invention sont avantageusement des copolymères triblocs du type ABA comportant deux blocs terminaux thermoplastiques A styrène et une séquence centrale élastomère B qui est une oléfine, éventuellement associés à des copolymères diblocs du type AB comportant un bloc thermoplastique A styrène et une séquence élastomère B qui est une oléfine. Les séquences B oléfines de ces copolymères peuvent être constituées d'oléfines insaturées comme par exemple isoprène ou butadiène ou d'oléfines saturées comme par exemple éthylène-butylène ou éthylène-propylène.

Dans le cas d'un mélange de copolymères triblocs ABA et de copolymères diblocs AB, on pourra employer des mélanges de copolymères triblocs ABA et de copolymères diblocs AB commerciaux déjà disponibles ou réaliser des mélanges en toute proportion préalablement choisie à partir de deux produits disponibles indépendamment.

Les copolymères triblocs à séquence centrale insaturée sont bien connus de l'homme de l'art et sont notamment commercialisés par la société KRATON POLYMERS sous la dénomination KRATON^{®} D.

Comme exemples de copolymères poly(styrène-isoprène-styrène) (en abrégé SIS) on peut ainsi citer les produits commercialisés sous les dénominations KRATON^{®} D1107 ou KRATON^{®} D1119 BT ou encore les produits commercialisés par la société EXXON MOBIL CHEMICAL sous la dénomination VECTOR^{®} comme par exemple le produit commercialisé sous la dénomination VECTOR^{®} 4113. Comme exemple de copolymères poly(styrène-butadiène-styrène) le produit commercialisé sous la dénomination KRATON^{®} D1102.

Comme exemples de mélanges commerciaux de copolymères triblocs ABA et diblocs AB dans lesquels B est de l'isoprène, on peut citer les produits commercialisés par la société EXXON MOBIL CHEMICAL sous la dénomination VECTOR^{®} 4114.

Tous ces copolymères à base d'isoprène ou de butadiène présentent généralement une teneur en styrène comprise entre 10 et 52% en poids rapportée au poids total dudit copolymère.

Dans le cadre de la présente invention, on préférera utiliser les copolymères séquencés triblocs poly(styrène-isoprène-styrène) (en abrégé SIS) ayant une teneur en styrène comprise entre 14 et 52 % et de préférence entre 14 et 30 % en poids rapporté au poids dudit poly(SIS).

D'une façon préférée, on utilisera pour réaliser les compositions de la présente invention des copolymères séquencés triblocs et en particulier le produit commercialisé par la société KRATON POLYMERS sous la dénomination KRATON^{®} D1119 BT.

Les copolymères triblocs à séquence centrale saturée sont également bien connus de l'homme de l'art et sont par exemple commercialisés :
- par la société KRATON POLYMERS sous la dénomination KRATON^{®} G, et en particulier sous la dénomination KRATON^{®} G1651, KRATON^{®} G1654 ou KRATON^{®} G1652 pour les copolymères séquencés poly(styrène-éthylène-butylène-styrène) (en abrégé SEBS);
- par la société KURARAY sous la dénomination SEPTON^{®} pour les copolymères séquencés poly(styrène-éthylène-propylène-styrène) (en abrégé SEPS).

Comme exemple de mélanges commerciaux de copolymères triblocs et diblocs, on peut citer le produit commercialisé par la société KRATON POLYMERS sous la dénomination KRATON^{®} G1657 dont la séquence oléfine est éthylène-butylène.

Comme exemple d'un mélange particulier de copolymères triblocs et diblocs que l'on peut réaliser dans le cadre de la présente invention, on peut citer le mélange :
- d'un SEBS triblocs, comme en particulier le produit commercialisé par la société KRATON POLYMERS sous la dénomination KRATON^{®} G1651 ; et
- d'un copolymère diblocs poly(styrène-oléfine) comme en particulier le poly(styrène-éthylène-propylène) commercialisé par la société KRATON POLYMERS sous la dénomination KRATON^{®} G1702.

Dans le cadre de la présente invention, on préférera les copolymères triblocs SEBS ou SEPS ayant une teneur en styrène comprise entre 25 et 45 % en poids par rapport au poids dudit SEBS ou SEPS. D'une façon préférée, on utilisera des copolymères séquencés triblocs et en particulier les produits commercialisés par la société KRATON POLYMERS sous les dénominations KRATON^{®} G1651 et KRATON^{®} G1654.

De façon générale, l'élastomère sera utilisé dans des quantités adaptées selon la nature saturée ou insaturée de la séquence centrale oléfine du copolymère séquencé. Ainsi dans le cas d'un copolymère triblocs à séquence centrale insaturée il sera utilisé en une quantité de l'ordre de 10 à 30 % en poids, de préférence de 10 à 20 % en poids, rapportée au poids total de la composition. Dans le cas d'un copolymère triblocs à séquence centrale saturée il sera utilisé en une quantité de l'ordre de 3 à 10 % en poids, de préférence de 4 à 7 % en poids, rapporté au poids total de la composition.

Par hydrocolloïde ou particules d'hydrocolloïde, on entend désigner ici tout composé habituellement utilisé par l'homme de l'art pour son aptitude à absorber les liquides aqueux tels que l'eau, le sérum physiologique ou les exsudats d'une plaie.

Comme hydrocolloïdes appropriés, on peut citer par exemple la pectine, les alginates, les gommes végétales naturelles comme en particulier la gomme de Karaya, les dérivés de cellulose tels que les carboxyméthylcelluloses et leurs sels de métal alcalin tels que le sodium ou le calcium, ainsi que les polymères synthétiques à base de sels de l'acide acrylique, connus sous l'appellation "superabsorbants", comme par exemple les produits commercialisés par la société BASF sous la dénomination LUQUASORB^{®} 1003 ou par la société CIBA Specialty Chemicals sous la dénomination SALCARE^{®} SC91 ainsi que les mélanges de ces composés.

Certains de ces superabsorbants qualifiés de « microcolloïdes » car ils présentent une taille de particules inférieure à 10 micromètres peuvent bien entendu être utilisée dans le cadre de la réalisation de la composition.

Les hydrocolloïdes préférés dans le cadre de la présente invention sont les sels de métal alcalin de la carboxyméthylcellulose, et en particulier la carboxyméthylcellulose de sodium (CMC). La taille des particules d'hydrocolloïdes est par exemple comprise entre 50 et 100 microns, notamment de l'ordre de 80 microns.

La quantité d'hydrocolloïdes incorporés dans la composition élastomère sera avantageusement de l'ordre de 2 à 20 % en poids, de préférence de 5 à 18 % en poids, de préférence encore de 8 à 18 % en poids, de préférence encore de 12 à 16 % en poids, rapportée au poids total de la composition élastomère. Les hydrocolloïdes, introduits en trop grande quantité dans une couche contact perforée, diminuent la capacité d'absorption d'un non tissé à base de fibres superabsorbantes au fur et à mesure que le gel se forme. En effet, la forte capacité d'absorption des hydrocolloïdes entraine un gonflement de la couche contact, si bien que les trous de la maille peuvent se boucher. Le non tissé absorbant n'absorbe plus directement les exsudats mais absorbe les exsudats présents dans la couche absorbante hydrocolloïde ce qui diminue la capacité d'absorption du pansement et crée des problèmes de macération.

Ladite composition comprend un ou plusieurs élastomères choisis parmi les polymères séquencés poly(styrène-oléfine-styrène) en association avec un ou plusieurs composés plastifiants et, si nécessaire, un ou plusieurs antioxydants.

Les compositions élastomère des pansements selon la présente invention comprennent un (ou plusieurs) composé(s) plastifiant(s) destinés à améliorer leurs propriétés d'étirement, de souplesse, d'extrudabilité ou de mise en oeuvre.

Il s'agira de préférence de composés liquides, compatibles avec la séquence centrale oléfine des copolymères séquencés utilisés.

Parmi les composés plastifiants susceptibles d'être utilisés à cet effet, on peut citer en particulier les huiles minérales plastifiantes, quelle que soit la nature de la séquence centrale. On peut également citer les polybutènes - comme par exemple les produits commercialisés par la société BP CHEMICALS sous la dénomination NAPVIS^{®} 10 -ou encore des dérivés de phtalate tels que le dioctylphtalate ou le dioctyladipate, lorsque la séquence centrale est insaturée.

Alternativement, on peut aussi utiliser des produits de synthèse à base de mélanges liquides d'hydrocarbures saturés comme par exemple les produits commercialisés par la société TOTAL sous la dénomination GEMSEAL^{®} et en particulier le produit GEMSEAL^{®} 60 qui est un mélange isoparaffinique issu d'une coupe pétrolière totalement hydrogénée. On utilisera de préférence ces produits avec un copolymère triblocs comportant une séquence centrale saturée.

Dans le cadre de la présente invention, on utilisera de préférence des huiles plastifiantes et en particulier des huiles minérales formées de composés de nature paraffinique, naphténique ou aromatique ou de leurs mélanges dans des proportions variables.

Parmi les huiles plastifiantes convenant particulièrement, on peut citer :
- les produits commercialisés par la société SHELL sous les dénominations ONDINA^{®} et RISELLA^{®} qui sont constitués de mélanges à base de composés naphténiques et paraffiniques ;
- les produits commercialisés sous la dénomination CATENEX^{®} qui sont constitués de mélanges à base de composés naphténiques, aromatiques et paraffiniques.

D'une façon particulièrement préférée, on utilisera une huile plastifiante minérale choisie parmi les produits commercialisés sous les dénominations ONDINA^{®}963 et ONDINA^{®}919.

Ces composés plastifiants peuvent être utilisés en une quantité de l'ordre de 20 à 65% en poids, de préférence de 30 à 50% en poids, rapportée au poids total de la composition élastomère hydrocolloïde.

Selon un mode de réalisation, ces compositions sont adhérentes : elles ont la propriété d'adhérer à la peau sans adhérer à la plaie. Elles comprennent un ou plusieurs composés dits « tackifiants » tels que ceux habituellement utilisés par l'homme de l'art dans la préparation des adhésifs sensibles à la pression à base d'élastomères. Pour une description détaillée de ces produits, on pourra se reporter à l'ouvrage de Donatas Satas "Handbook of Pressure Sensitive Technology", 3rd Edition, 1999, pages 346 à 398.

Dans le cadre de la présente invention on préférera l'utilisation d'une couche contact qui possède un faible pouvoir adhérent. Ce faible pouvoir adhérent permet en effet au personnel soignant de disposer de ses deux mains une fois le pansement posé, afin de poser des éléments secondaires comme par exemple des bandes de contention voire de repositionner le pansement sans altérer les tissus sains. De tels pansements sont qualifiés de micro-adhérents.

D'une façon générale, on pourra utiliser un (ou plusieurs) produit(s) tackifiant(s) qui sera (seront) incorporés(s) à la matrice élastomérique dans une proportion de l'ordre de 1 à 50 % en poids, rapportée au poids total de la composition élastomère hydrocolloïde, qui sera déterminée en fonction de la nature et de la proportion relative des autres constituants de cette dernière, pour atteindre le pouvoir micro-adhérent souhaité pour le pansement.

De préférence, le(s) produit(s) tackifiant(s) représentera (représenteront) de 10 à 45 % en poids, et de préférence encore de 15 à 40 % en poids du poids total de la composition élastomère hydrocolloïde.

Les produits tackifiants susceptibles d'être utilisés dans le cadre de la présente invention pourront être choisis parmi les résines tackifiantes, les polyisobutylènes de bas poids moléculaire ou leurs mélanges.

Parmi les résines tackifiantes susceptibles d'être utilisées selon l'invention, on peut mentionner les résines polyterpènes ou terpènes modifiées, les résines de colophane, les résines hydrocarbonnées, les mélanges de résines cycliques, aromatiques et aliphatiques, ou des mélanges de ces résines.

De tels produits sont commercialisés par exemple :
- par la société ARAKAWA Chemical Industries sous la dénomination ARKON^{®} P qui sont des résines polycyclopentadiénes hydrogénées ;
- par la société EXXON Chemical sous la dénomination ESCOREZ^{®} et en particulier la série des résines 5000 qui sont hydrogénées.
- par la société GOODYEAR sous la dénomination WINGTACK^{®}, et en particulier WINGTACK^{®} 86 qui est une résine de synthèse formée de copolymères en C5/C9 ou WINGTACK^{®} 10 qui est une résine à base de polyterpène synthétique ;
- par la société HERCULES sous la dénomination KRISTALEX^{®} et en particulier KRISTALEX^{®} 3085 qui est une résine à base d'alpha-méthylstyrène.

De façon générale, afin d'éviter les problèmes de coloration et de stabilité des résines insaturées, on préférera l'utilisation de résines hydrogénées, en particulier avec les copolymères triblocs à séquence centrale saturée car elles sont beaucoup plus compatibles avec ces derniers que les résines insaturées type WINGTACK que l'on utilise essentiellement avec les copolymères triblocs à séquence centrale insaturée.

Parmi ces dernières on préférera utiliser les résines ESCOREZ^{®} de la série 5000 et tout particulièrement la résine ESCOREZ^{®} 5380.

Les résines tackifiantes peuvent être utilisées seules ou en mélange avec d'autres produits tackifiants, de préférence dans une proportion de 10 à 50 % en poids, et plus particulièrement de 15 à 40 % en poids, rapportée au poids total de la composition.

Parmi les polyisobutylènes de bas poids moléculaire susceptibles d'être utilisés comme produits tackifiants on peut citer les polyisobutylènes ayant un poids moléculaire de l'ordre de 40 000 à 80 000 daltons, comme par exemple les produits commercialisés par la société BASF sous la dénomination OPPANOL^{®} et en particulier les produits commercialisés sous les dénominations OPPANOL^{®}B12 et OPPANOL^{®}B15 ou par la société EXXON Chemical sous la dénomination Vistanex et en particulier le grade LM-MH .

Ces polyisobutylènes pourront être utilisés seuls ou en mélange avec d'autres tackifiants en association avec les copolymères triblocs à séquence centrale insaturée. Leur proportion pourra varier dans ce cas entre 5 à 30 % en poids, et plus particulièrement de 8 à 15 % en poids, rapportée au poids total de la composition.

Les compositions susceptibles d'être utilisées pour la fabrication de pansements conformes à l'invention peuvent en outre comprendre, un ou plusieurs agents antioxydants.

Par agents antioxydants, on entend désigner ici les composés couramment employés par l'homme de l'art pour assurer la stabilité des composés entrant dans la formulation des masses adhésives, en particulier les résines tackifiantes et les copolymères séquencés, vis-à-vis de l'oxygène, la chaleur, l'ozone et les rayonnements ultra-violets.

Comme exemples d'agents antioxydants appropriés, on peut citer :
- les antioxydants phénoliques comme en particulier les produits commercialisés par la société CIBA SPECIALTY CHEMICALS sous les dénominations IRGANOX^{®} 1010, IRGANOX^{®} 565, IRGANOX^{®} 1076 ;
- les antioxydants soufrés comme en particulier le dibutyldithiocarbamate de zinc commercialisé par la société AKZO sous la dénomination PERKACIT^{®} ZDBC.

Ces antioxydants pourront être utilisés en une quantité de l'ordre de 0,05 à 1 % en poids, de préférence de 0,1 à 0,5 % en poids, rapportée au poids total de la composition élastomère.

Dans le cadre de la présente invention, on préférera l'utilisation des produits IRGANOX^{®} et en particulier du produit IRGANOX^{®} 1010.

Divers composés peuvent en outre être ajoutés dans la formulation des compositions élastomères comme en particulier des adjuvants ou des agents actifs couramment utilisés dans le domaine du traitement des plaies ou dans le domaine pharmacologique.

La composition peut contenir des principes actifs ayant un rôle favorable dans le traitement de la plaie. Ces principes actifs peuvent notamment induire ou accélérer la cicatrisation en agissant pendant la phase de détersion et/ou de granulation de la plaie. On a pu observer que la couche contact de l'invention est un très bon vecteur du relargage du ou des principes actifs, notamment grâce à la présence des hydrocolloïdes.

Ces agents actifs pourront être utilisés en une quantité de l'ordre de 0,01 à 20 % en poids, de préférence de 1 à 15 % en poids et en particulier de 2 à 10 % en poids rapportée au poids total de la composition.

Parmi les substances actives susceptibles d'être utilisées dans le cadre de l'invention, on peut citer, à titre d'exemples, des agents bactéricides ou bactériostatiques, des agents favorisant la cicatrisation, des agents anti-douleurs ou des agents anti-inflammatoires.

Comme adjuvants, on peut ainsi citer des matières colorantes, des charges, des absorbeurs ou piégeurs d'odeurs, des filtres UV, des régulateurs de pH, des microcapsules ou des microsphères qui peuvent éventuellement contenir des agents actifs , de la vaseline pour donner au pansement un aspect gras ou des polymères ou des tensioactifs pour optimiser la vitesse de gélification, de mouillabilité ou le relargage des actifs de la composition.

Lorsque la composition contient des polymères insaturés, on peut ainsi utiliser le copolymère AcResin^{®} pour augmenter la gélification ; on peut également utiliser le tensioactif MONTANOX^{®} 80 ou le polymère SEPINOV^{®} EMT 10 tous les deux commercialisés par la société SEPPIC pour optimiser la vitesse de gélification, la mouillabilité ou le relargage d'actifs éventuellement présents dans la composition.

Dans le cadre de la réalisation de pansements qui utilisent une composition à base de copolymères élastomères à séquence centrale saturée, on préférera employer des compositions qui, pour un total de 100 % en poids, comprennent :
0,05 à 1 % en poids d'antioxydant ;
10 à 50 % en poids de résine tackifiante ;
2 à 20 %, de préférence de 12 à 16 %, en poids d'hydrocolloïde, et en particulier de carboxyméthylcellulose de sodium ;
20 à 65 % en poids de plastifiant, et en particulier d'une huile minérale plastifiante ;
3 à 10 % en poids d'un polymère tribloc poly(styrène-éthylène-butylène-styrène) ou poly(styrène- éthylène- propylène- styrène) ;
1 à 15 % en poids d'un copolymère constitué d'un sel de l'acide 2-méthyl-2-[(1-oxo-2-propényl)amino]-1-propane-sulfonique et de l'ester 2-hydroxyéthyle de l'acide propénoïque.

Dans le cadre de la réalisation de pansements qui utilisent une composition à base de copolymères élastomères à séquence centrale saturée, on préférera employer des compositions qui, pour un total de 100 % en poids, comprennent :
0,05 à 1 % en poids d'antioxydant ;
10 à 50 % en poids de résine tackifiante ;
2 à 20 %, de préférence de 12 à 16 %, en poids d'hydrocolloïde, et en particulier de carboxyméthylcellulose de sodium ;
20 à 65 % en poids de plastifiant, et en particulier d'une huile minérale plastifiante ;
3 à 10 % en poids d'un polymère tribloc poly(styrène-éthylène-butylène-styrène) ou poly(styrène- éthylène- propylène- styrène) ;

Une autre composition à base de copolymères élastomères à séquence centrale saturée, pourra comprendre, pour un total de 100 % en poids:
0,05 à 1 % en poids d'antioxydant ;
2 à 20 %, de préférence de 12 à 16 %, en poids d'hydrocolloïde, et en particulier de carboxyméthylcellulose de sodium ;
20 à 65 % en poids de plastifiant, et en particulier d'une huile minérale ;
3 à 25 % en poids d'un polymère tribloc poly(styrène-éthylène-butylène-styrène) ou poly(styrène-éthylène-propylène-styrène).

Dans le cadre de la réalisation des pansements qui utilisent une composition à base de copolymères élastomères à séquence centrale insaturée, on préférera employer des compositions qui, pour un total de 100 % en poids, comprennent :
0,05 à 1 % en poids d'antioxydant ;
10 à 60 % en poids de résine tackifiante ;
2 à 20 %, de préférence de 12 à 16 %, en poids d'hydrocolloïde, et en particulier de carboxyméthylcellulose de sodium ;
10 à 65 % en poids de plastifiant, et en particulier d'une huile minérale ou d'un dérivé de phtalate ;
5 à 25 % en poids d'un polymère tribloc poly(styrène-butadiène-styrène) ou poly(styrène- isoprène - styrène);
1 à 15 % en poids d'un copolymère constitué d'un sel de l'acide 2-méthyl-2-[(1-oxo-2-propényl)amino]-1-propane-sulfonique et de l'ester 2-hydroxyéthyle de l'acide propénoïque.

Une composition particulièrement préférée comprend, pour un total de 100 % en poids:
0,05 à 1 % en poids d'antioxydant ;
30 à 40 % en poids de résine tackifiante ;
2 à 20 %, de préférence de 12 à 16 %, en poids d'hydrocolloïde, et en particulier de carboxyméthylcellulose de sodium ;
35 à 45 % en poids de plastifiant, et en particulier d'une huile minérale plastifiante ;
4 à 6 % en poids d'un polymère tribloc poly(styrène-éthylène-butylène-styrène) ou poly(styrène- éthylène- propylène- styrène);
2 à 8 % en poids d'un copolymère constitué d'un sel de l'acide 2-méthyl-2-[(1-oxo-2-propényl)amino]-1-propane-sulfonique et de l'ester 2-hydroxyéthyle de l'acide propénoïque.

Les compositions définies précédemment peuvent être fabriquées suivant un procédé hot melt bien connu de l'homme du métier, par malaxage à chaud des différents constituants à une température comprise entre 90 et 160°C et de préférence entre 110 et 140°C. On préfère déposer la composition élastomère hydrocolloïde par trempage d'un cylindre gravé dans la composition préalablement malaxée à chaud. La composition ainsi moulée est ensuite transférée par le cylindre sur le non tissé.

De façon pratique, la composition élastomère sera protégée en la recouvrant au moins sur sa face destinée à venir en contact avec la plaie, d'une couche ou pellicule de protection, qui pourra être retirée par pelage avant utilisation du pansement.

La couche contact comprenant la composition élastomère décrite précédemment peut prendre différentes formes telles qu'une feuille de composition élastomère perforée, un filet de composition extrudé ou moulé, ou un tricot enduit de ladite composition élastomère. Pour obtenir l'adhérence requise, une couche contact plane sera préférée.

La taille des ouvertures de la couche contact est de préférence choisie de telle sorte que leur diamètre moyen ou leur longueur moyenne soit comprise entre 1 et 3 mm, de préférence entre 1 et 2 mm, notamment de l'ordre de 1,5 mm. La surface des ouvertures est par exemple comprise entre 0,5 et 10 mm², de préférence entre 0,8 et 6 mm².

Selon un mode de réalisation, la couche contact ne recouvre pas plus de 80% de ladite surface pour permettre une absorption optimale des exsudats par la compresse. La couche contact est configurée avantageusement de telle sorte qu'elle recouvre, avant d'être exposée aux exsudats de la plaie, entre 50 et 80%, de préférence entre 65 et 75%, de la surface de la compresse venant en regard de la plaie.

Le grammage de la couche contact va de préférence de 110 à 250 g/m², de préférence encore de 160 à 200 g/m². Il est par exemple de l'ordre de 185 g/m². La Demanderesse propose donc une couche contact de grammage élevé et qui recouvre une grande proportion de la surface de la compresse. Contre toute attente, cette couche contact n'entrave pas la vitesse et la capacité d'absorption des exsudats par la compresse.

Selon un mode de mise en oeuvre, la couche contact a la forme d'un filet de fils dont l'épaisseur - mesurée dans le plan parallèle à sa plus grande surface - est comprise entre 1 et 3 mm, et dont l'espacement est compris entre 1 et 3 mm.

Dans un mode de réalisation de l'invention, le grammage de la compresse et le grammage de la couche contact sont sensiblement identiques. L'écart entre les deux grammages est par exemple inférieur à 20%, de préférence encore inférieur à 10%, par rapport à la valeur du grammage le plus élevé.

La taille, la forme et la disposition relative des ouvertures de la couche contact sont choisies de telle manière que ladite couche soit suffisamment résistante aux déformations imposées pendant son application sur la compresse non tissée, par exemple pendant une étape de démoulage à chaud. Les ouvertures doivent aussi être suffisamment grandes et les fils de compositions doivent être suffisamment fins pour que la compresse puisse absorber les exsudats plus rapidement que les hydrocolloïdes dispersés dans la couche contact.

Un mode de réalisation particulier de l'invention est tel que ladite couche :
- est sous la forme d'un filet de fils dont l'épaisseur des fils - mesurée dans le plan parallèle à sa plus grande surface - est comprise entre 1 et 3 mm, et dont l'espacement entre les fils est compris entre 1 et 3 mm,
- présente un grammage allant de 170 à 200 g/m² et
- comprend de 12 à 16% en poids d'hydrocolloïdes par rapport au poids de la composition qui constitue la couche contact.

Les caractéristiques qui ont été décrites précédemment en rapport avec la présente invention s'appliquent bien entendu à ce mode de réalisation particulier. Par exemple, le grammage de la compresse peut être sensiblement égal à celui de la couche contact.

On préfèrera que le procédé d'enduction de la compresse par la couche contact utilise une étape de transfert sur cylindre gravé. Le cylindre vient baigner dans la composition élastomère fondue, avant de démouler la trame encore chaude sur la compresse non tissée. Ce procédé permet avantageusement d'incorporer des particules hydrocolloïdes solides de granulométrie assez élevée. L'application de la composition encore chaude sur la compresse permet en outre d'optimiser l'accroche et la couche contact sur la compresse.

L'invention est illustrée par l'exemple suivant.

### Exemple 1 :

### Préparation du non tissé

On a préparé un non tissé de 185 g/m² et de 2 mm d'épaisseur à l'aide de fibres superabsorbantes LANSEAL^{®} F commercialisées par la société TOYOBO CO LTD, et de fibres thermoliantes bicomposants polyester/polyéthylène, dans un ratio massique 70% (fibres superabsorbantes) / 30% (fibres thermoliantes).

Les fibres sont pesées, mélangées, cardées, puis nappées pour obtenir un voile de fibres. Une opération d'aiguilletage permet ensuite de consolider ce voile. La consolidation finale du non tissé se fait par chauffage (calandrage) afin de faire fondre l'écorce des fibres thermoliantes et bloquer le non tissé dans sa configuration finale.

### Préparation de la masse élastomère hydrocolloïde

On a par ailleurs préparé une composition élastomère hydrocolloïde par mélange dans un malaxeur.

La composition élastomère, exprimée en pourcentage en poids par rapport au poids total de la composition était la suivante :
- Huile minérale commercialisée par la société Shell sous la dénomination Ondina ^{®}919 : 41,7%
- Sel de sodium de carboxyméthylcellulose (hydrocolloïde) commercialisé par la société AQUALON sous la dénomination CMC Blanose ^{®}7H4XF : 14,8%
- Copolymère séquencé de poly(styrène-éthylène-butylène-styrène) (élastomère) commercialisé par la société KRATON sous la dénomination KRATON^{®} G 1651 E : 4,7%
- Antioxydant commercialisé sous la dénomination IRGANOX^{®} 1010 par la société CIBA SPECIALTY CHEMICALS : 0,2%
- Résine tackifiante commercialisée par la société EXXON CHEMICALS sous la dénomination ESCOREZ^{®} 5380 : 35,6%
- Copolymère de sel de l'acide 2-méthyl-2[(1-oxo-2-propényl)amino]-1-propanesulfonique et de l'ester 2-hydroxyéthyle de l'acide propénoïque commercialisé par la société SEPPIC sous la dénomination SEPINOV^{®} EMT 10 : 5%.

On a introduit les différents constituants à une température comprise entre 105 et 115°C sous agitation, de manière à obtenir un mélange homogène.

Plus précisément, on a introduit initialement l'huile minérale, l'hydrocolloïde, et l'élastomère puis l'antioxydant, le SEPINOV^{®} EMT 10 et enfin la résine tackifiante.

### Enduction du non tissé avec la masse adhésive

Cet adhésif a été enduit sur le non tissé à un grammage de 180 g/m² sous la forme d'un filet dont la maille est carrée. L'enduction est réalisée par transfert hot melt sur cylindre gravé. La largeur des fils est de 1,6 mm. Les ouvertures carrées ont une surface de 4 mm². La surface recouverte est de 70 %. L'épaisseur des fils de la masse adhésive est de 0,2 mm.

### Test du retrait de la matrice de fibrine in vitro :

Les matrices de fibrine ont été préparées selon le protocole de Brown décrit dans la publication "Fibroblast migration in fibrin gel matrices" Arm J. Pathol, 1993, 142 : 273-283.

Les composants et le mode opératoire qui ont été utilisé sont les suivants :
On a solubilisé à 37°C :
   - 5 ml d'une solution aqueuse comprenant 50 millimoles d'Hépès (Catalogue Sigma-Aldrich)
   - 15 mg de fibrinogène de plasma humain (catalogue Sigma-Aldrich)
   - 5 millimoles de CaCl₂.

A la solution ainsi préparée, on a ajouté 50 µl de thrombine, 100 NIH de plasma humain (catalogue Sigma-Aldrich).

L'ensemble a été déposé dans une boîte de Pétri puis laissé à incuber à 37°C pendant 24 heures.

Au bout de 24 heures, la matrice de fibrine est formée. Un échantillon de pansement fabriqué comme précédemment est déposé sur la matrice, à température ambiante, pendant 24 heures.

Au retrait, on observe que la fibrine a été décrochée du support et se trouve transférée en un seul bloc à la surface du pansement que l'on a retiré.

### Exemples 2 et 3 comparatifs ;

### Préparation du non tissé

La préparation du non tissé des exemples comparatifs 2 et 3 est identique à celle décrite dans l'exemple 1.

### Composition de la masse élastomère hydrocolloïde de l'exemple 2 comparatif

La composition élastomère de l'exemple 2 comparatif était identique à celle décrite dans l'exemple 1

### Composition de la masse élastomère hydrocolloïde de l'exemple 3 comparatif

La composition élastomère, exprimée en pourcentage en poids par rapport au poids total de la composition était la suivante :
- Huile minérale commercialisée par la société Shell sous la dénomination Ondina ^{®}917 : 32,62%
- Sel de sodium de carboxyméthylcellulose (hydrocolloïde) commercialisé par la société AQUALON sous la dénomination CMC Blanose ^{®}7H4XF : 30%
- Copolymère séquencé de poly(styrène-éthylène-butylène-styrène) (élastomère) commercialisé par la société KRATON sous la dénomination KRATON^{®} G 1651 E : 3,86%
- Antioxydant commercialisé sous la dénomination IRGANOX^{®} 1010 par la société CIBA SPECIALTY CHEMICALS : 0,16%
- Résine tackifiante commercialisée par la société EXXON CHEMICALS sous la dénomination ESCOREZ^{®} 5380 : 25%
- Copolymère de sel de l'acide 2-méthyl-2[(1-oxo-2-propényl)amino]-1-propanesulfonique et de l'ester 2-hydroxyéthyle de l'acide propénoïque commercialisé par la société SEPPIC sous la dénomination SEPINOV^{®} EMT 10 : 11%.

### Préparation de la masse élastomère hydrocolloïde des exemples 2 et 3 comparatifs

La préparation de la masse élastomère hydrocolloïde des exemples comparatifs est identique à celle décrite dans l'exemple 1.

### Enduction du non tissé avec la masse adhésive

Pour l'exemple 2 comparatif l'adhésif a été enduit sur le non tissé à un grammage de 662 g/m² sous la forme d'un filet dont la maille est carrée. L'enduction a été réalisée par transfert hot melt sur cylindre gravé. L'épaisseur des fils était de 0,84 mm.

Pour l'exemple 3 comparatif l'adhésif a été enduit sur le non tissé à un grammage de 840 g/m² sous la forme d'un filet dont la maille est carrée. L'enduction a été réalisée par transfert hot melt sur cylindre gravé. L'épaisseur des fils était de 1 mm.

### Mesure de la capacité d'absorption :

La mesure de la capacité d'absorption a été réalisée selon la norme EN ISO 9073-12 relative à la mesure de la capacité d' absorption d'un non tissé, à la différence près que l'on a utilisé une plaque en verre poreux de 30 mm de diamètre et que l'ensemble pièce en mousse hydrophobe/poids a été remplacé par une plaque en plexiglas/poids qui applique au global une pression de 40 mm de mercure.

Le liquide utilisé était une solution de NaCl / CaCl₂ qui comprenait 298 g de NaCl et 368 g de CaCl₂ par litre d'eau.

Les résultats d'absorption exprimés en grammes ont été reportés dans le tableau ci-dessous.

| Produits | Masse de solution NaCl/CaCl2 absorbée au bout de 1h (g) | Masse de solution NaCl/CaCl2 absorbée au bout de 24h (g) |
|---|---|---|
| Exemple 1 | 3,016 | 11,976 |
| Exemple 2 comparatif | 0,459 | 8,847 |
| Exemple 3 comparatif | 0,364 | 8,348 |

Le pansement de l'exemple 2 comparatif dont le grammage de la masse élastomère hydrocolloïde est supérieur à 500 g/m2 absorbe - à 1 heure et à 24 heures - beaucoup moins que le pansement de l'exemple 1 de l'invention bien que le taux d'hydrocolloïdes soit identique.

Le pansement de l'exemple 3 comparatif dont le grammage de la masse élastomère hydrocolloïde est supérieur à 500 g/m2 et dont le taux d'hydrocolloïde est supérieur à 20% en poids absorbe - à 24 heures - moins que le pansement de l'exemple 1 de l'invention. L'utilisation de la couche contact spécifique de l'exemple 1 démontre que l'on ne ralentit pas la vitesse d'absorption du non tissé et que cette capacité d'absorption est conservée au cours du temps contrairement aux couches contacts définies dans les contre-exemples.

## Revendications

1. Pansement comprenant :
a- une compresse non tissée absorbante formée d'un mélange de fibres superabsorbantes bicomposants et de fibres non absorbantes thermoliantes, l'ensemble des fibres étant thermolié,
et,
b- une couche contact recouvrant partiellement la face de la compresse destinée à venir en contact avec la plaie, ladite couche comprenant des ouvertures permettant le passage des exsudats de la plaie et présentant un grammage allant de 110 à 500 g/m², et ladite couche étant formée d'une composition comprenant une matrice élastomérique et des hydrocolloïdes, la proportion d'hydrocolloïdes étant comprise entre 2 et 20% en poids du poids de ladite composition.

2. Pansement selon la revendication 1, **caractérisé en ce que** les fibres non absorbantes thermoliantes sont bicomposants.

3. Pansement selon la revendication 2, **caractérisé en ce que** les fibres non absorbantes thermoliantes sont bicomposants du type coeur/écorce, ledit coeur étant en polyéthylène téréphtalate et l'écorce étant en polyéthylène.

4. Pansement selon l'une des revendications précédentes, **caractérisé en ce que** les fibres superabsorbantes sont bicomposants de type coeur/écorce, ledit coeur étant en polyacrylonitrile et l'écorce en étant en polyacrylate.

5. Pansement selon l'une des revendications précédentes, **caractérisé en ce que** le grammage de la compresse va de 40 à 400 g/m².

6. Pansement selon l'une des revendications précédentes, **caractérisé en ce que** l'épaisseur de la compresse va de 0,6 à 3 mm.

7. Pansement selon l'une des revendications précédentes, **caractérisé en ce que** le ratio massique entre les fibres absorbantes et les fibres non absorbantes thermoliantes est compris entre 60/40 et 80/20.

8. Pansement selon l'une des revendications précédentes, **caractérisé en ce que** le grammage de la couche contact va de 150 à 200 g/m².

9. Pansement selon l'une des revendications précédentes, **caractérisé en ce que** la couche contact, avant d'être exposée aux exsudats de la plaie, recouvre entre 50 et 80%, de préférence entre 65 et 75% de la face de la compresse qui est en regard de la plaie.

10. Pansement selon l'une des revendications précédentes, **caractérisé en ce que** la couche contact est sous la forme d'un filet de fils dont l'épaisseur - mesurée dans le plan parallèle à sa plus grande surface - est comprise entre 1 et 3 mm, et dont l'espacement est compris 1 et 3 mm.

11. Pansement selon l'une des revendications précédentes, **caractérisé en ce que** les ouvertures de la couche contact ont une surface comprise entre 0,5 et 10 mm², de préférence comprise entre 0,8 et 6 mm².

12. Pansement selon l'une des revendications précédentes, **caractérisé en ce que** les hydrocolloïdes sont choisis parmi les sels de métal alcalin de carboxyméthylcellulose.

13. Pansement selon l'une des revendications précédentes, **caractérisé en ce que** les hydrocolloïdes représentent de 8 à 18% en poids par rapport au poids total de la composition.

14. Pansement selon l'une des revendications précédentes, **caractérisé en ce que** les hydrocolloïdes représentent de 12 à 16% en poids par rapport au poids total de la composition.

15. Pansement selon l'une des revendications précédentes, **caractérisé en ce que** la couche contact contient un actif qui favorise la cicatrisation ou le traitement de la plaie.

## Patentansprüche

1. Wundverband, umfassend:
a- eine absorbierende Vlieskompresse, die von einer Mischung aus superabsorbierenden Zweikomponentenfasern und nicht absorbierenden wärmeverbindbaren Fasern gebildet ist, wobei die Gesamtheit der Fasern wärmeverbunden ist,
und
b- eine Kontaktschicht, die teilweise die Seite der Kompresse bedeckt, die dazu bestimmt ist, mit der Wunde in Kontakt zu kommen, wobei die Schicht Öffnungen umfasst, die den Durchgang des Wundsekrets ermöglichen, und ein Flächengewicht von 110 bis 500 g/m² aufweist, und wobei die Schicht von einer Zusammensetzung, umfassend eine Elastomermatrix und Hydrokolloide, gebildet ist, wobei der Hydrokolloidanteil zwischen 2 und 20 Gew.-% des Gewichts der Zusammensetzung beträgt.

2. Wundverband gemäß Anspruch 1, **dadurch gekennzeichnet, dass** die nicht absorbierenden wärmeverbindbaren Fasern Zweikomponentenfasern sind.

3. Wundverband gemäß Anspruch 2, **dadurch gekennzeichnet, dass** die nicht absorbierenden wärmeverbindbaren Fasern Zweikomponentenfasern vom Typ Kern/Schale sind, wobei der Kern aus Polyethylenterephthalat und die Schale aus Polyethylen ist.

4. Wundverband gemäß einem der vorhergehenden Ansprüche, **dadurch gekennzeichnet, dass** die superabsorbierenden Fasern Zweikomponentenfasern vom Typ Kern/Schale sind, wobei der Kern aus Polyacrylnitril und die Schale aus Polyacrylat ist.

5. Wundverband gemäß einem der vorhergehenden Ansprüche, **dadurch gekennzeichnet, dass** das Flächengewicht der Kompresse 40 bis 400 g/m² beträgt.

6. Wundverband gemäß einem der vorhergehenden Ansprüche, **dadurch gekennzeichnet, dass** die Dicke der Kompresse 0,6 bis 3 mm beträgt.

7. Wundverband gemäß einem der vorhergehenden Ansprüche, **dadurch gekennzeichnet, dass** das Massenverhältnis zwischen den absorbierenden Fasern und den nicht absorbierenden wärmeverbindbaren Fasern zwischen 60/40 und 80/20 beträgt.

8. Wundverband gemäß einem der vorhergehenden Ansprüche, **dadurch gekennzeichnet, dass** das Flächengewicht der Kontaktschicht 150 bis 200 g/m² beträgt.

9. Wundverband gemäß einem der vorhergehenden Ansprüche, **dadurch gekennzeichnet, dass** die Kontaktschicht, bevor sie dem Wundsekret ausgesetzt wird, zwischen 50 und 80 %, vorzugsweise zwischen 65 und 75 %, der Fläche der Kompresse, die vor der Wunde liegt, bedeckt.

10. Wundverband gemäß einem der vorhergehenden Ansprüche, **dadurch gekennzeichnet, dass** die Kontaktschicht in Form eines Netzes von Fäden vorhanden ist, dessen Dicke - gemessen in der Ebene parallel zu seiner größten Oberfläche-zwischen 1 und 3 mm beträgt, und dessen Abstand zwischen 1 und 3 mm beträgt.

11. Wundverband gemäß einem der vorhergehenden Ansprüche, **dadurch gekennzeichnet, dass** die Öffnungen der Kontaktschicht eine Oberfläche zwischen 0,5 und 10 mm², vorzugsweise zwischen 0,8 und 6 mm², aufweist.

12. Wundverband gemäß einem der vorhergehenden Ansprüche, **dadurch gekennzeichnet, dass** die Hydrokolloide unter den Alkalimetallsalzen von Carboxymethylzellulose ausgewählt sind.

13. Wundverband gemäß einem der vorhergehenden Ansprüche, **dadurch gekennzeichnet, dass** die Hydrokolloide 8 bis 18 Gew.-% bezogen auf das Gesamtgewicht der Zusammensetzung darstellen.

14. Wundverband gemäß einem der vorhergehenden Ansprüche, **dadurch gekennzeichnet, dass** die Hydrokolloide 12 bis 16 Gew.-% bezogen auf das Gesamtgewicht der Zusammensetzung darstellen.

15. Wundverband gemäß einem der vorhergehenden Ansprüche, **dadurch gekennzeichnet, dass** die Kontaktschicht einen Wirkstoff enthält, der die Wundheilung oder die Behandlung der Wunde begünstigt.

## Claims

1. A dressing comprising:
a- an absorbent nonwoven compress formed from a mixture of bicomponent superabsorbent fibers and of thermal bonding non-absorbent fibers, all of the fibers being thermally bonded,
and,
b- a contact layer that partially covers the face of the compress intended to come into contact with the wound, said layer comprising openings that allow the passage of exudates from the wound and having a basis weight that ranges from 110 to 500 g/m², and said layer being formed from a composition comprising an elastomeric matrix and hydrocolloids, the proportion of hydrocolloids being between 2% and 20% by weight of the weight of said composition.

2. The dressing as claimed in claim 1, **characterized in that** the thermal bonding non-absorbent fibers are bicomponent fibers.

3. The dressing as claimed in claim 2, **characterized in that** the thermal bonding non-absorbent fibers are bicomponent fibers of core/shell type, said core being made of polyethylene terephthalate and the shell being made of polyethylene.

4. The dressing as claimed in one of the preceding claims, **characterized in that** the superabsorbent fibers are bicomponent fibers of core-shell type, said core being made of polyacrylonitrile and the shell being made of polyacrylate.

5. The dressing as claimed in one of the preceding claims, **characterized in that** the basis weight of the compress ranges from 40 to 400 g/m².

6. The dressing as claimed in one of the preceding claims, **characterized in that** the thickness of the compress ranges from 0.6 to 3 mm.

7. The dressing as claimed in one of the preceding claims, **characterized in that** the weight ratio between the absorbent fibers and the thermal bonding non-absorbent fibers is between 60/40 and 80/20.

8. The dressing as claimed in one of the preceding claims, **characterized in that** the basis weight of the contact layer ranges from 150 to 200 g/m².

9. The dressing as claimed in one of the preceding claims, **characterized in that** the contact layer, before being exposed to the exudates of the wound, covers between 50% and 80%, preferably between 65% and 75%, of the face of the compress which is opposite the wound.

10. The dressing as claimed in one of the preceding claims, **characterized in that** the contact layer is in the form of a net fabric of yarns, the thickness of which - measured in the plane parallel to its greatest surface area - is between 1 and 3 mm, and the spacing of which is between 1 and 3 mm.

11. The dressing as claimed in one of the preceding claims, **characterized in that** the openings of the contact layer have a surface area between 0.5 and 10 mm², preferably between 0.8 and 6 mm².

12. The dressing as claimed in one of the preceding claims, **characterized in that** the hydrocolloids are chosen from alkali metal salts of carboxymethyl cellulose.

13. The dressing as claimed in one of the preceding claims, **characterized in that** the hydrocolloids represent from 8% to 18% by weight relative to the total weight of the composition.

14. The dressing as claimed in one of the preceding claims, **characterized in that** the hydrocolloids represent from 12% to 16% by weight relative to the total weight of the composition.

15. The dressing as claimed in one of the preceding claims, **characterized in that** the contact layer contains an active agent which promotes the healing or the treatment of the wound.
